# EUROPEAN PATENT SPECIFICATION

(11) **EP 0 993 291 B1**
(45) Date of publication and mention of the grant of the patent: **08.10.2003**
(21) Application number: 98932672.3
(22) Date of filing: 18.06.1998
(51) Int. Cl.: A61F 13/15

(54) **ABSORBENT ARTICLE AND METHOD FOR PRODUCING SAME**
ABSORBIERENDE ARTIKEL UND VERFAHREN ZUR HERSTELLUNG
ARTICLE ABSORBANT ET PROCEDE DE PRODUCTION

(30) Priority: 02.07.1997 SE 9702545
(43) Date of publication of application: 19.04.2000
(73) Proprietor: SCA Hygiene Products AB, 405 03 Göteborg (SE)
(72) Inventor: SANDIN, Cécile, S-431 36 Mölndal (SE); GRÄNSBO, Göran, S-433 62 Sävedalen (SE)
(74) Representative: Romare, Laila Anette
(86) International application number: SE9801184
(87) International publication number: WO99001098

(56) References cited:
- US-A- 3 262 451
- US-A- 3 306 795
- US-A- 4 326 528

## Description

### TECHNICAL FIELD:

The invention relates to a disposable product used as an absorbent article, such as an incontinence protector, diaper, sanitary towel or the like, having an essentially stable cup shape, and to a method for producing such an article.

### STATE OF THE ART:

Absorbent articles of the type mentioned above generally present an elongate shape and have two longitudinal side edges, two transverse end edges and also a back portion, a front portion and an intermediate crotch portion. The article includes a liquid-permeable surface layer directed towards the user during use, a liquid-impermeable surface layer directed away from the user during use, and an absorbent body arranged between the layers.

It is often desirable for an absorbent article to have a cup-shaped portion, at least around the wet point, for receiving excreted body fluids, since such a cup-shaped portion reduces the risk of leakage along the side edges of the article.

One method for producing a cup-shape in an absorbent article is to use elastic members, generally in the form of elastic filaments, or bands. In order for the article to obtain a curved shape, elastic members are disposed in at least one direction on the absorbent article, and the elastic members are in most cases stretched out. After applying the elastic members, the elastic-coated article web is generally kept stretched since subsequent process stages may necessitate a flat article web. As the elastic members are sometimes disposed in a direction other than the direction of travel of the web and in addition are stretched out, such manufacturing processes demand, inter alia, that the elastic members rapidly adhere to the article web. A manufacturing process with a stretched out elastic web which must adhere rapidly to an article web therefore entails often complicated technical solutions.

In order, inter alia, to avoid problems which can arise with stretched-out elastic members which are to be applied across or along the direction of travel of an article web at high speeds, heat-shrinkable elastic materials can be used. For example, in European Patent 429,265 (Eaton), a stretched plastic film is applied to the waist portions of the article. The waist portions are then exposed to heat and the film shrinks to its original shape and becomes elastic. In US 4,917,682 (Lancaster, Urban), heat-shrinkable elastic materials are used both on the waist portions of the article and also along the side portions of the article. When these portions are heated, the heat-shrinkable materials contract, forming leg elastics and waist elastics.

A disadvantage of absorbent articles made with elastic is that they can pull too tightly around the user's body when used, on account of the contraction of the elastic members, and this can cause skin irritation. Because the elastic members in a relaxed state may have pulled the article together more than is desirable, stretching the article out upon use may cause excessive tightening around the body, which can lead to skin irritation. An article made of elastic members is not dimensionally stable either, and instead the shape depends on the degree of stretching or contraction of the elastic. An article which is not dimensionally stable can be deformed upon use on account of the user's movements, and leakage along the side edges of the article and/or the end edges of the article can then occur.

Articles can obtain a stable cup shape by being formed in moulds, as in WO 95/15736 (Melius et al.) for example. The liquid-impermeable surface layer is pre-formed by heating to its melt point, after which a final shape is obtained by vacuum-forming against an inner. surface of the mould. Remaining material is then applied over the pre-formed liquid-impermeable surface layer. However, articles which are formed by means of three-dimensionally curved pre-formed material being included in the article cannot be manufactured using a flat web, since the articles are already curved at the start of the manufacturing process. Application of the remaining material in a continuous process cannot therefore take place without complicated process solutions. The document US 3,306,795 discloses an absorbent article and method of producing the same. The article is provided with a heat-shrunk outer layer, generating a generally arcuately shaped article.

Nonelastic materials included in an absorbent article may also, depending on how they are designed, give an absorbent article a three-dimensionally curved shape. For example, an inner liquid-permeable surface layer can have a surface area which is smaller than the surface area of the liquid-impermeable surface layer. Attaching tie surface layers to each other, for example edge to edge, means that the article acquires a curved three-dimensional shape which is virtually stable. The disadvantage is that it is complicated, in one manufacturing process, to join together two materials of different sizes, where one material upon application has a greater surface area between the points of attachment than does the other one.

Laminates of materials of different surface sizes can be included in an absorbent article so that the article will obtain a corrugated surface, which gives a three-dimensional shape to the surface. Such laminates can include nonwoven material, plastic material, etc. To obtain a three-dimensional laminate, material with shrinkability can be used, inter alia. One layer of the laminate should in this case have a greater shrinkability than other layers included in the laminate. On shrinkage, a corrugated surface is thus obtained in which material with low shrinkability buckles between points of attachment to material with high shrinkability. Such laminates are generally pre-shrunk before they are applied to an absorbent article so that their shape is not altered at subsequent process stages. Nonwoven materials including a mixture of fibres of different shrinkability can also, by shrinking, obtain a three-dimensional structure or other properties, such as elasticity. It is not normally desirable, for example, for a surface layer of a nonwoven material containing shrink fibres to have shrinkability upon or after application of the material to an absorbent article, and for this reason such materials have been pre-shrunk before application.

### OBJECTS:

One object of the present invention is to make available a virtually dimensionally stable article. Another object of the invention is to be able to shape an article without the web of material having to be kept stretched during manufacture. Moreover, as a result of the invention, it is possible to control the final shape of the article and to give the article a virtually stable cup shape. Articles according to the invention can be manufactured continuously in a flat web of material. A further object of the invention is to be able to use the shrink properties of the material included in order to shape the article into a cup shape and in this way to use material which does not need to be pre-shrunk before being applied.

### DESCRIPTION OF THE INVENTION:

In accordance with the invention, an article of the type discussed in the introduction has been obtained, comprising a liquid-permeable surface layer directed towards the user during use, a liquid-impermeable surface layer directed away from the user during use, and an absorbent body arranged between the layers. The invention also relates to a method for shaping such an article. The article according to the invention considerably reduces the problems associated with elastic cup-shaped articles and is in addition easier to manufacture than previously known dimensionally stable articles. The article comprises at least one heat-shrinkable material which has been heat-treated after application of the material to the article, as a result of which the material has been made to shrink in one or more directions by means of exposure to heat, a first surface of the article, intended to be directed towards a user during use, having an essentially stable cup shape which creates a space between a user and the article for receiving urine and/or excreta.

The invention also relates to a method for manufacturing an article having a cup shape. The method is distinguished by the fact that a heat-shrinkable material is attached to the article, after which the article is exposed to heat, as a result of which the heat-shrinkable material included in the article is made to shrink, so that the surface of the article intended to be directed towards a user during use has a virtually stable cup shape. Prior to the manufacture of the article, the heat-shrinkable material must not have been heat-treated in a manner which has completely or to a significant extent destroyed the shrinkability of the material.

By applying one or more materials in at least one direction on the article, which materials have a greater shrinkability than other materials included in the article, the article can be shaped by heating. During heating, the materials which have a greater shrinkability shrink in at least one direction, as a result of which a cup-shaped article is obtained. By using material which shrinks upon exposure of the article to heat, it is possible in a simple way to create an article with a cup shape and to avoid the aforementioned problems which arise when it is necessary to handle stretched-out material webs in a manufacturing process.

By using materials which do not become appreciably elastic through shrinkage, the article acquires a virtually stable shape. Because the article has a virtually stable shape, the said shape is not appreciably affected by the movements of a user.

An article with a cup shape, as described here, is understood to mean an article in which a liquid-receiving side of the article has a surface curved in towards the article for receiving excreta and/or urine, that side of the article which, upon use, is directed towards a user having side portions which are raised towards the user from a plane surface through the article. An inwardly curved surface can, for example, be formed as an elongate trench with raised longitudinal side portions, or as a pocket, or with some other shape which creates a space, between a user and the article, for receiving urine and/or excreta.

By applying to the article two or more materials with greater shrinkability than the other material included in the article, but with different degrees of shrinkability, it is possible to obtain different degrees of contraction of the article.

The degree of shrinkage can also be controlled, inter alia, by the temperature which is used for heating the article and by the time for which the article is exposed to heat.

The heat-shrinkable material can advantageously be applied to flat article webs. The heat-shrinkable material can be attached by adhesive bonding, ultrasonic welding, etc., to the liquid-impermeable surface layer, the absorbent core or the liquid-permeable surface layer. Alternatively, the heat-shrinkable material can be attached to two or more materials included in the article. The article web can then be kept flat through subsequent process stages, for example coating of the liquid-permeable surface layer if the heat-shrinkable material is to be placed on that side of the liquid-permeable surface layer which is directed away from the user's body.

The heat-shrinkable material can also replace the liquid-permeable surface layer, and the article can in that case be shaped by means of the whole surface layer being allowed to shrink either by exposing the whole article to heat or, through oriented heat exposure, by shrinking only selected parts of the surface layer. The heat-shrinkable material can be included as a layer in a surface layer laminate and, at the same time as the article obtains a cup shape, a surface layer with a folded surface can then also be obtained. The heat-shrinkable material can be perforated so as to make it liquid-permeable.

A material with high shrinkability can also be shrunk at the same time as it is being attached to the article, for example by welding. By gradually increasing the temperature, the material continues to shrink after it has been attached to the absorbent article and the article then acquires its virtually stable cup shape. The heat-shrinkable material is expediently placed across the surface of the absorbent core which is directed towards a user's body so that a cup will be formed.

The article is expediently shaped after joining the inner liquid-permeable surface layer and the outer liquid-impermeable surface layer. The article is preferably heated in a final stage of the manufacturing process so that the web can be kept flat throughout the entire process.

The shrinkability of the material can also be affected by the way in which the material is attached to the other material included in the article. If the heat-shrinkable material is, for example, attached at points to another material included in the article, the distance between the attachment points can influence the degree of shrinkage since the heat-shrinkable material has a lower shrinkability at the attachment, points. Material with small distances between the attachment points therefore shrinks less than material with a greater distance between the attachment points. The same also applies to other patterns of attachment, for example an attachment pattern consisting of filaments and obtained, for example, from filaments of adhesive, where the space between the attachment areas formed influences the degree of shrinkability. Such filaments are not necessarily straight, or disposed in one direction, but can be disposed in curves, spirals, in different directions, in different patterns, etc.

The heat-shrinkable material can also be applied to the article in different patterns in order to adapt the shape of the article to a wearer. One or more strips and/or filaments of heat-shrinkable material can in this case be applied to the article in one or more directions. The heat-shrinkable material is expediently placed across that surface of the absorbent core which, during use, is directed towards the user's body.

After all the coating of the material included in the article, the article web can be heated by means of air streams oriented towards the article web. Heat treatment can of course also be carried out using ultrasound, infrared heat, or some other type of heat radiation. The articles can also be directly exposed to heat transfer, by heat transfer from at least one contact surface, such as, for example, a heated conveyor roller or a heated conveyor belt. A combination of heat radiation and heat convection is also possible, for example the articles can be heated in ovens.

By using heat-shrinkable material, it is possible to obtain an improved fit, distance between the article and a user's body, sealing between the side edges of the article and the user's body, cavity space for excreta and/or urine, and folding of the article.

The articles do not need to be heated along the production path, and can instead obtain their cup shape by means of after-treatment. It is also possible to shape the articles by heating packs which contain the articles.

The heat-shrinkable material can comprise fibres which do not shrink, which means that the material obtains a soft and airy structure after shrinkage. A raised edge, or an edge seam formed by shrunk material, which bears against a user's body can also be made soft by coating or folding a soft material along the edge, for example cotton wool, soft nonwoven or foam.

The heat-shrinkable material can be shrinkable in one or more directions of shrinking.

A combination of heat-shrinkable material and elastic members in the article is possible. The article is then provided with heat-shrinkable material in order to obtain a virtually stable cup shape, while elastic members can contribute to achieving a good fit and leaktightness. For example, elastic members can be applied along the longitudinal side edges of an article in order to obtain so-called leg elastic. The article then obtains, by means of the heat-shrinkable material not having been pre-shrunk, a stable three-dimensionally curved shape, while the elastic members contribute to providing a sealing effect against a user's thighs. Elastic members can also be applied along the end edges of the article, providing sealing against the user's waist. Other combinations are of course also possible.

The fact that the article has a virtually stable shape means that the article will not be stretchable to any appreciable extent, but that it will preferably be able to be folded or pressed together so as to be easily packaged.

Suitable materials for forming an article according to the present invention are materials. which shrink when the material reaches a temperature above 40°C. It is more advantageous to use materials which shrink when the material reaches a temperature above 50°C and preferably above 55°C. The risk of such a material shrinking at the temperatures the material may be exposed to during transport and storage is in fact very small. The temperature which the heat-shrinkable material reaches during the heat treatment should not exceed 130°C and preferably should not exceed 100°C.

Suitable heat-shrinkable materials are materials with a maximum shrinkability of 2-80%, preferably 5-50%, at the temperature used. After exposure to heat, the material should have an extent, in the direction of shrinkage, which is 2-80% and preferably 5-50% smaller than the original extent. Those materials which are shrinkable at a relatively low temperature upon shaping of the absorbent article should expediently be allowed to shrink to their maximum shrinkability. This prevents the materials from being affected, after shaping, by the surrounding temperature in such a way that the shape obtained upon manufacture of the absorbent article is lost.

Heat-shrinkable materials which are suitable for application according to the invention are, for example, polyolefin-based shrink film, polyolefin-based nonwoven which has been treated as a polyolefin-based shrink film so that the material will be shrinkable, or a polyester-based nonwoven which has not been heat-treated, as a result of which the polyester fibres have retained their shrinkability. Before application, the materials must not have been heat-treated in a manner which has completely or to a significant extent removed the shrinkability of the material.

### BRIEF DESCRIPTION OF THE FIGURES:

The invention will now be described in greater detail with reference to the attached figures, where:
Figure 1 is a diagrammatic representation of an incontinence protector according to a first illustrative embodiment of the invention,
Figure 2 is a diagrammatic representation of an incontinence protector according to a second illustrative embodiment of the invention, and
Figure 3 is a diagrammatic representation of an incontinence protector according to a third illustrative embodiment of the invention.

### ILLUSTRATIVE EMBODIMENTS:

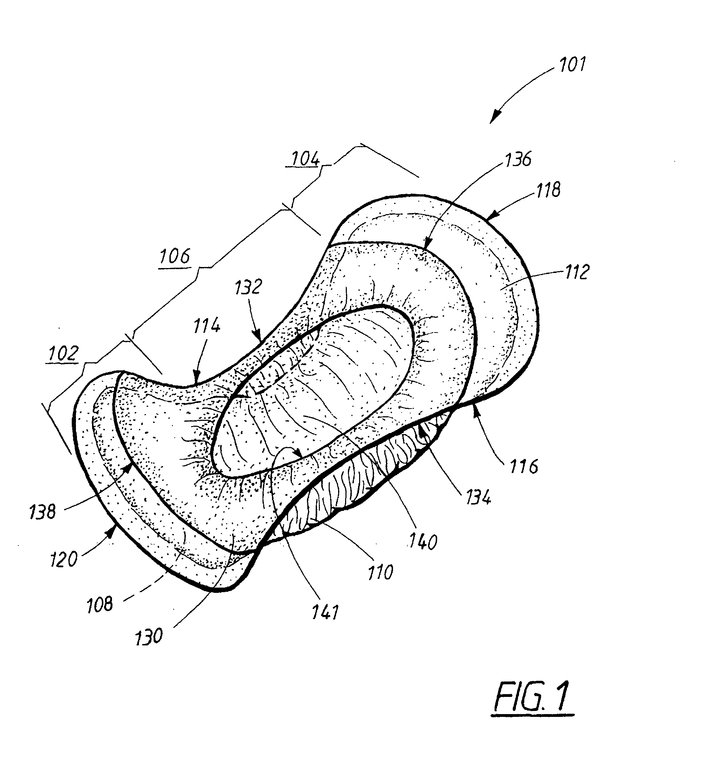

The incontinence protector 101 shown in Figure 1 is divided into a back portion 102, a front portion 104 and an intermediate crotch portion 106. The incontinence protector comprises an absorbent body 108, enclosed between a liquid-impermeable surface layer 110 which, during use, is intended to be directed towards the user's undergarments, and a liquid-permeable surface layer 112 which is intended to be directed towards the user's body. The liquid-impermeable surface layer 110 and the liquid-permeable surface layer 112 are connected to each other along portions which extend outside the absorbent body 108, all round the latter.

The incontinence protector moreover has two longitudinal side edges 114, 116 and two transverse end edges 118, 120. The distance between the two longitudinal side edges 114, 116 is shorter at the intermediate crotch portion 106 than the distance between these at the end edges 118, 120 of the incontinence protector, as a result of which the side edges 114, 116 of the incontinence protector obtain drawn-in central portions adapted to the shape of the user's thighs.

In the embodiment shown in Figure 1, a heat-shrinkable material 130 is placed across the intermediate crotch portion 106. The width of the material is almost the same as the width of the underlying incontinence protector, as a result of which the longitudinal side edges 132, 134 of the material coincide with the longitudinal side edges 114, 116 of the incontinence protector. The length of the material is less than the length of the incontinence protector, as a result of which the transverse end edges 136, 138 of the material are situated at a distance from the transverse end edges 118, 120 of the incontinence protector. Along its longitudinal side edges 132, 134, the material 130 has been connected to the inner surface layer 112 along portions which extend along the longitudinal side edges 114, 116 of the incontinence protector, while at its transverse end edges 136, 138 it has been connected to the inner surface layer 112 in portions overlying the absorbent body 108.

Between the connected transverse and longitudinal edges 136, 138; 132, 134, a hole 140 has been cut out or punched out from the heat-shrinkable material 130. Upon heating of the incontinence protector 101, the heat-shrinkable material 130 shrinks and draws together between the edges 132, 134, 136, 138 and the edge 141 around the hole 140, as a result of which the incontinence protector 101 obtains a cup shape around the wet point, and as a result of which the non-connected heat-shrinkable material 130 forms barriers, raised from the absorbent barrier 108, around the hole 140. Below the hole 140 and its barriers formed by shrinkage, a cup is formed for receiving excreta and urine. Excreta and urine passing through the hole towards the absorbent body 108 are in this way kept at a distance from the user's body.

According to an alternative embodiment, the heat-shrinkable material 130 in the non-shrunk state can have the same extent as the underlying incontinence protector 101. The heat-shrinkable material 130 is in this case attached at least along portions extending outside the absorbent body 108, all around the latter. The material with shrinkability 130 can also have a width which is smaller than the width of the incontinence protector. Its side edges 132, 134 are then attached to the underlying surface layer 112 outside or above the absorbent body 108.

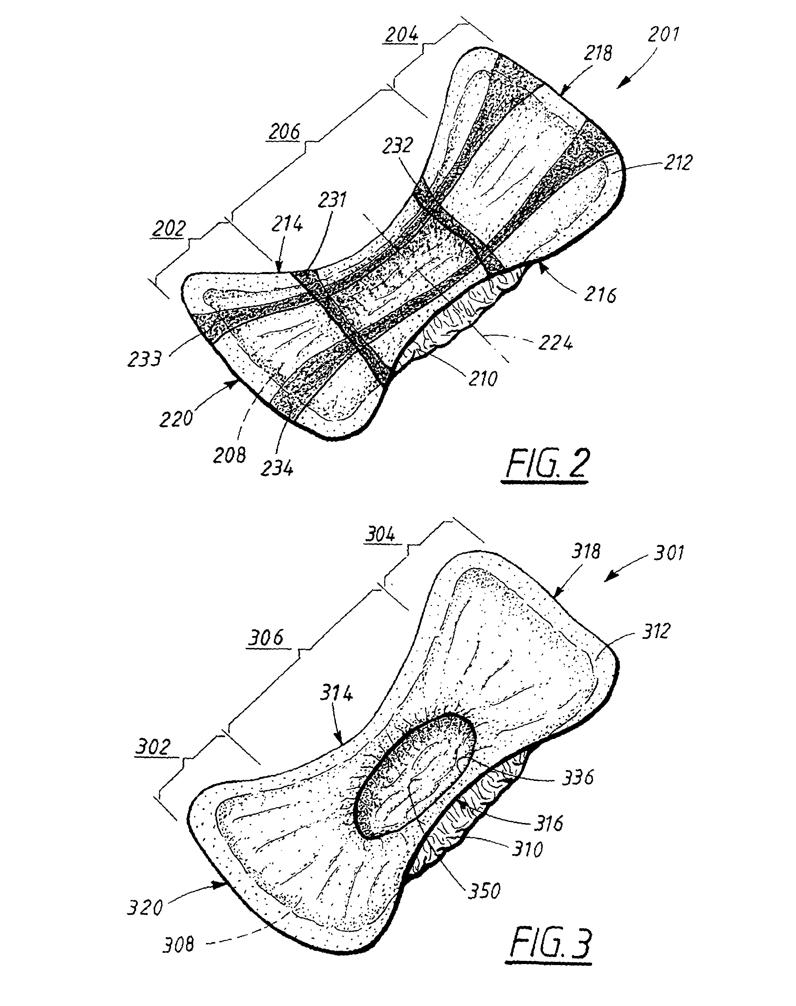

The incontinence protector 201 shown in Figure 2 is divided into a back portion 202, a front portion 204 and an intermediate crotch portion 206. The incontinence protector is made up of an absorbent body 208, enclosed between a liquid-impermeable surface layer 210 and a liquid-permeable surface layer 212 which is intended to be directed towards a user's body during use. The liquid-impermeable surface layer 210 and the liquid-permeable surface layer 212 are connected to each other along portions which extend outside the absorbent body 208, all round the latter. The incontinence protector moreover has two longitudinal side edges 214, 216 and two transverse end edges 218, 220. The distance between the two longitudinal side edges 214, 216 is shorter at the intermediate crotch portion 206 than the distance between these at the end edges 218, 220 of the incontinence protector, as a result of which the side edges 214, 216 of the incontinence protector obtain drawn-in central portions adapted to the shape of the user's thighs.

In Figure 2, transverse strips 231, 232 of a heat-shrinkable material have been placed on each side of a transverse centre line 224 through the incontinence protector 201. The transverse strips 231, 232 are arranged over the absorbent body 208 and extend from one longitudinal side edge 214 of the incontinence protector to the other longitudinal side edge 216 of the incontinence protector. Longitudinal strips 233, 234 of a heat-shrinkable material have been placed along the longitudinal side edges 214, 216 of the incontinence protector. Both the longitudinal strips 233, 234 and the transverse strips 231, 232 have been attached to the liquid-permeable surface layer 212 on that side of the surface layer 212 which is directed away from the user's body. Upon heating of the incontinence protector, both the longitudinal and the transverse strips shrink. The shrinkage of the longitudinal strips 233, 234 means that the incontinence protector curves in the longitudinal direction, as a result of which the longitudinal side edges 214, 216 of the incontinence protector are lifted from a plane surface through the protector. The shrinkage of the transverse strips 231, 232 means that the incontinence protector curves in a transverse direction. Of course, strips can be arranged in only one direction, if so desired.

The incontinence protector 301 shown in Figure 3 is divided into a back portion 302, a front portion 304 and an intermediate crotch portion 306. The incontinence protector is made up of an absorbent body 308, enclosed between a liquid-impermeable surface layer 310 and a liquid-permeable surface layer 312 which is intended to be directed towards a user's body during use. The outer surface layer 310 and the inner surface layer 312 are connected to each other along portions which extend outside the absorbent body 308, all round the latter.

The incontinence protector 301 moreover has two longitudinal side edges 314, 316 and two transverse end edges 318, 320. The distance between the two longitudinal side edges 314, 316 is shorter at the intermediate crotch portion 306 than the distance between these at the end edges 318, 320 of the incontinence protector, as a result of which the side edges 314, 316 of the incontinence protector obtain drawn-in central portions adapted to the shape of the user's thighs.

In Figure 3, a filament 336 of heat-shrinkable material has been disposed in the crotch portion 306 of the incontinence protector, in a curve formed as an oval and attached to the liquid-permeable surface layer 317. Upon shrinkage of the filament 336, the surface layer 312 bearing against the filament 336 draws together, as a result of which a cup-shaped portion 350 is obtained inside the filament for receiving excreta and urine. Excreta and urine which is collected in the cup-shaped portion 350 can thus be kept at a distance from the user's skin.

Instead of filament, a strip of a heat-shrinkable material can of course be used. Alternatively, such a cup shape can be obtained by means of the liquid-permeable surface layer comprising shrink fibres and hot air jets being directed at the article, for example along the periphery of an oval.

Transverse end edges and longitudinal side edges of an article do not mean that these edges need to be linear or parallel to a transverse or longitudinal axis through the article. The transverse end edges are intended to bear against a wearer's waist area upon use, while the longitudinal edges are intended to provide a seal against a wearer's thighs. The longitudinal side edges can, for example at the intermediate crotch portion, be curved in towards a longitudinal centre line through the article, forming inwardly curved portions for following the shape of a wearer's thighs. The longitudinal edges can also comprise at least one portion curved inwards, parallel to the transverse end edges of the article, towards a longitudinal centre line through the axis forming a T shape. The article can have any shape within the range known, for example hourglass shape, rectangular, T shape, etc.

Filaments and strips of heat-shrinkable material can of course be disposed in patterns or directions other than those which have been described in the three above illustrative embodiments for forming the article. For example, strips or filaments of heat-shrinkable material can be disposed to form a V shape, with the tip of the V at a longitudinal centre line through the article. Heat-shrinkable material can also be applied to the article in a zigzag pattern.

Alternatively, the inner liquid-permeable surface layer can be a shrinkable nonwoven material. The cup shape of the article is then obtained by directing hot streams of air at those portions of the liquid-permeable surface layer where shrinkage is desired. The whole of the inner surface layer can also be exposed to heating in order to shape the article. A surface layer which is not connected to the absorbent body can be shrunk to produce a cavity between the surface layer and the absorbent core.

The invention must not be seen as being limited to the illustrative embodiments described here, and instead further variants and modifications are possible within the scope of the appended patent claims. For example, the invention is naturally suitable for types of absorbent articles other than the incontinence protectors described in the illustrative embodiments.

## Claims

1. Absorbent article, such as an incontinence protector, a diaper, a sanitary towel or the like, having a back portion (102; 202; 302), a front portion (104; 204; 304) and an intermediate crotch portion (106; 206; 306), and the article comprising a liquid-permeable surface layer (112; 212; 312) arranged on a first surface of the article, and a liquid-impermeable surface layer (110; 210; 310) arranged on a second surface of the article, and an absorbent body (108; 208; 308) arranged between the layers (112, 110; 212, 210; 312, 310), the absorbent article comprising at least one heat-shrinkable material (130; 231, 232, 233, 234; 336) which extends over the first surface of the article and permits passage of fluid into the article, and which has been heat-treated after application of the material to the article, as a result of which the material has been made to shrink in one or more directions by means of exposure to heat, **characterized in that** the first surface of the article has an essentially stable cup shape which creates a space between a user and the article for receiving urine and/or excreta.

2. Absorbent article according to Claim 1, **characterized in that** the liquid-permeable surface layer (112; 212; 312) is made of a heat-shrinkable material.

3. Absorbent article according to Claim 1 or 2, **characterized in that** it comprises a heat-shrinkable nonwoven material.

4. Absorbent article according to any of Claims 1, 2 or 3, **characterized in that** it comprises a heat-shrinkable shrink film.

5. Absorbent article according to any of the preceding claims, **characterized in that** a heat-shrinkable material (130), having a hole (140), is placed over the crotch portion (106).

6. Absorbent article according to any of the preceding claims, **characterized in that** the article comprises at least one strip (231, 232, 233, 234) of a heat-shrinkable material, disposed in one or more directions.

7. Absorbent article according to any of the preceding claims, **characterized in that** the article comprises at least one filament (336) of a heat-shrinkable material disposed in one or more directions.

8. Absorbent article according to any of the preceding claims, **characterized in that** the material with shrinkability is shrinkable in at least one direction of shrinking, the material, after exposure to heat, having an extent in the direction of shrinking which is 2-80% and preferably 5-50% smaller than the original extent.

9. Method for forming an article, such as an incontinence protector, a diaper, a sanitary towel or the like, having a back portion (102; 202; 302), a front portion (104; 204; 304) and an intermediate crotch portion (106; 206; 306), a liquid-permeable surface layer (112; 212; 312) being arranged on a first surface of the article, and a liquid-impermeable surface layer (110; 210; 310) being arranged on a second surface of the article, and an absorbent body (108; 208; 308) being arranged between the layers (112, 110; 212, 210; 312, 310), wherein a heat-shrinkable material (130; 231, 232, 233, 234; 336) which permits passage of fluid into the article, is attached to the first surface of the article, after which the article is exposed to heat, as a result of which the heat-shrinkable material is made to shrink, **characterized in that** the first surface of the article is given a virtually stable cup shape which creates a space between a user and the article for receiving urine and/or excreta.

10. Method for forming an absorbent article according to Claim 9, **characterized in that** the heat exposure is effected using oriented air streams, ovens, ultrasound or infrared heat.

11. Method for forming an absorbent article according to any of Claims 9 or 10, **characterized in that** the heat exposure is effected using at least one hot contact surface.

12. Method for forming an absorbent article according to Claim 9, 10 or 11, **characterized in that** the heat exposure is effected after packaging the articles.

## Patentansprüche

1. Absorptionsartikel, wie z.B. Inkontinenzschutz, Windel, Hygienebinde oder ähnliches mit einem Rückabschnitt (102; 202; 302), einem Vorderabschnitt (104; 204; 304) und einem Zwischen-Schrittabschnitt (106; 206; 306), und wobei der Artikel eine flüssigkeitsdurchlässige Oberflächenschicht (112; 212; 312), die an einer ersten Oberfläche des Artikels angeordnet ist, und eine flüssgkeitsundurchlässige Oberflächenschicht (110; 210; 310), die an einer zweiten Oberfläche des Artikels angeordnet ist, und einen Absorptionskörper (108; 208; 308) aufweist, der zwischen den Schichten (112, 110; 212, 210; 312, 310) angeordnet ist, wobei der Absorptionsartikel wenigstens ein wärmeschrumfbares Material (130; 231, 232, 233, 234; 336) aufweist, das sich über die erste Oberfläche des Artikels erstreckt und einen Durchgang von Fluid in den Artikel ermöglicht, und das nach der Aufbringung des Materials an den Artikel wärmebehandelt wurde, wobei als Ergebnis dessen das Material veranlasst wurde, in einer oder mehreren Richtungen mittels Wärme-Aussetzen zu schrumpfen,
**dadurch gekennzeichnet, dass**
die erste Oberfläche des Artikels eine im Wesentlichen stabile Napfform aufweist, die einen Raum zwischen einem Benutzer und dem Artikel zur Aufnahme von Urin und/oder Exkrementen schafft.

2. Absorptionsartikel nach Anspruch 1,
**dadurch gekennzeichnet, dass**
die flüssigkeitsdurchlässige Oberflächenschicht (112; 212; 312) aus einem wärmeschrumpfbaren Material ausgeführt ist.

3. Absorptionsartikel nach Anspruch 1 oder 2,
**dadurch gekennzeichnet, dass**
er ein wärmeschrumpfbares, nicht gewebtes Material aufweist.

4. Absorptionsartikel nach einem der Ansprüche 1, 2 oder 3,
**dadurch gekennzeichnet, dass**
er eine wärmeschrumpfbare Schrumpffolie aufweist.

5. Absorptionsartikel nach einem der vorangehenden Ansprüche,
**dadurch gekennzeichnet, dass**
ein wärmeschrumpfbares Material (130) mit einer Öffnung (140) über den Schrittabschnitt (106) angeordnet ist.

6. Absorptionsartikel nach einem der vorangehenden Ansprüche,
**dadurch gekennzeichnet, dass**
der Artikel wenigstens einen Streifen (231, 232, 233, 234) aus einem wärmeschrumpfbaren Material aufweist, das in einer oder mehreren Richtungen angeordnet ist.

7. Absorptionsartikel nach einem der vorangehenden Ansprüche,
**dadurch gekennzeichnet, dass**
der Artikel wenigstens einen Faden (336) aus wärmeschrumpfbarem Material aufweist, das in einer oder mehreren Richtungen angeordnet ist.

8. Absorptionsartikel nach einem der vorangehenden Ansprüche,
**dadurch gekennzeichnet, dass**
das Material mit Schrumpffähigkeit in wenigstens einer Schrumpfrichtung schrumpfbar ist, wobei das Material nach dem Wärme-Aussetzen eine Erstreckung in der Schrumpfrichtung aufweist, die 2-80% und vorzugsweise 5-50% kleiner als der Ausgangszustand ist.

9. Verfahren zur Ausbildung eines Artikels, wie z.B. eines Inkontinenzschutzes, einer Windel, einer Hygienebinde oder ähnlichem mit einem Rückabschnitt (102; 202; 302), einem Vorderabschnitt (104; 204; 304) und einem Zwischen-Schrittabschnitt (106; 206; 306), wobei eine flüssigkeitsdurchlässige Oberflächenschicht (112; 212; 312) an einer ersten Oberfläche des Artikels angeordnet ist, und eine flüssigkeitsundurchlässige Oberflächenschicht (110; 210; 310) an einer zweiten Oberfläche des Artikels angeordnet ist, und ein Absorptionskörper (108; 208; 308) zwischen den Schichten (112, 110; 212, 210; 312, 310) angeordnet ist, wobei ein wärmeschrumpfbares Material (130; 231, 232, 233, 234; 336), das den Durchgang von Fluid in den Artikel ermöglicht, an die erste Oberfläche des Artikels angebracht ist, woraufhin der Artikel Wärme ausgesetzt wird, und als Ergebnis dessen das wärmeschrumpfbare Material veranlasst wird, zu schrumpfen,
**dadurch gekennzeichnet, dass**
der ersten Oberfläche des Artikels eine nahezu stabile Napfform gegeben wird, die einen Raum zwischen einem Benutzer und dem Artikel zur Aufnahme von Urin und/oder Exkrementen erzeugt.

10. Verfahren zur Ausbildung eines Absorptionsartikels nach Anspruch 9,
**dadurch gekennzeichnet, dass**
die Wärme-Aussetzung unter Verwendung von gerichteten Luftströmen, Öfen, Ultraschall oder Infrarotwärme bewirkt wird.

11. Verfahren zur Ausbildung eines Absorptionsartikels nach einem der Ansprüche 9 oder 10,
**dadurch gekennzeichnet, dass**
die Wärme-Aussetzung unter Verwendung wenigstens einer heißen Berührungsfläche bewirkt wird.

12. Verfahren zur Ausbildung eines Absorptionsartikels nach Anspruch 9, 10 oder 11,
**dadurch gekennzeichnet, dass**
die Wärme-Aussetzung nach dem Verpacken der Artikel bewirkt wird.

## Revendications

1. Article absorbant, tel qu'une protection contre l'incontinence, une couche-culotte, une serviette hygiénique ou similaire, comportant une partie arrière (102 ; 202 ; 302), une partie avant (104 ; 204 ; 304) et une partie intermédiaire d'entrejambe (106 ; 206 ; 306), et l'article comprenant une couche de surface (112 ; 212 ; 312) perméable aux liquides agencée sur une première surface de l'article, et une couche de surface (110 ; 210; 310) imperméable aux liquides agencée sur une deuxième surface de l'article, et un corps absorbant (108 ; 208 ; 308) placé entre les couches (112, 110; 212, 210 ; 312, 310), l'article absorbant comprenant au moins un matériau thermorétractable (130 ; 231, 232, 233, 234 ; 236) qui s'étend sur la première surface de l'article et permet le passage de fluides entrant dans l'article, et qui a été traité par la chaleur après application du matériau sur l'article, en conséquence de quoi l'on a fait se rétrécir le matériau dans une ou plusieurs directions par exposition à la chaleur, **caractérisé en ce que** la première surface de l'article a une forme de coupe essentiellement stable qui crée un espace entre un utilisateur de l'article et l'article pour recevoir de l'urine et/ou des excreta.

2. Article absorbant selon la revendication 1, **caractérisé en ce que** la couche de surface (112 ; 212 ; 312) perméable aux liquides est faite d'un matériau thermorétractable.

3. Article absorbant selon la revendication 1 ou 2, **caractérisé en ce qu'**il comprend un matériau non tissé thermorétractable.

4. Article absorbant selon l'une quelconque des revendications 1 à 3, **caractérisé en ce qu'**il comprend un film thermorétractable.

5. Article absorbant selon l'une quelconque des revendications précédentes, **caractérisé en ce qu'**un matériau thermorétractable (130), comportant un trou (140) est placé sur la partie d'entrejambe (106).

6. Article absorbant selon l'une quelconque des revendications précédentes, **caractérisé en ce que** l'article comprend au moins une bande (231, 232, 233, 234) en matériau thermorétractable, disposée dans une ou plusieurs directions.

7. Article absorbant selon l'une quelconque des revendications précédentes, **caractérisé en ce que** l'article comprend au moins un filament (336) en matériau thermorétractable disposé dans une ou plusieurs directions.

8. Article absorbant selon l'une quelconque des revendications précédentes, **caractérisé en ce que** le matériau rétractable est rétractable dans au moins une direction de rétraction, le matériau, après exposition à la chaleur, ayant une dimension dans la direction de rétraction qui est de 2 à 80 % et de préférence de 5 à 50 % inférieure à la dimension d'origine.

9. Procédé de formation d'un article, tel qu'une protection contre l'incontinence, une couche-culotte, une serviette hygiénique ou similaire, comportant une partie arrière (102 ; 202 ; 302), une partie avant (104 ; 204 ; 304) et une partie intermédiaire d'entrejambe (106 ; 206 ; 306), une couche de surface (112 ; 212 ; 312) perméable aux liquides étant agencée sur une première surface de l'article, et une couche de surface (110 ; 210 ; 310) imperméable aux liquides étant agencée sur une deuxième surface de l'article, et un corps absorbant (108 ; 208 ; 308) étant placé entre les couches (112, 110 ; 212, 210 ; 312, 310), dans lequel un matériau thermorétractable (130 ; 231, 232, 233, 234 ; 236) qui permet le passage de fluides entrant dans l'article, est fixé à la première surface de l'article, après quoi l'article est exposé à la chaleur, en conséquence de quoi l'on fait se rétrécir le matériau thermorétractable, **caractérisé en ce que** l'on donne à la première surface de l'article une forme de coupe virtuellement stable qui crée un espace entre un utilisateur et l'article pour recevoir de l'urine et/ou des excreta.

10. Procédé de formation d'un article absorbant selon la revendication 9, **caractérisé en ce que** l'exposition à la chaleur est effectuée en utilisant des flux d'air orientés, des fours, des ultrasons ou de la chaleur par infrarouges.

11. Procédé de formation d'un article absorbant selon l'une quelconque des revendications 9 et 10, **caractérisé en ce que** l'exposition à la chaleur est effectuée en utilisant au moins une surface de contact chaude.

12. Procédé de formation d'un article absorbant selon la revendication 9, 10 ou 11, **caractérisé en ce que** l'exposition à la chaleur est effectuée après avoir emballé les articles.
